# EUROPEAN PATENT APPLICATION

(11) **EP 2 327 427 A1**
(43) Date of publication of application: **01.06.2011**
(21) Application number: 09808098.9
(22) Date of filing: 20.02.2009
(51) Int. Cl.: A61L 27/00, A61K 35/12

(54) **CELL PREPARATION FOR BONE TISSUE REGENERATION**

(30) Priority: 19.08.2008 JP 2008210591
(71) Applicant: Osaka University, Suita-shi Osaka 565-0871 (JP)
(72) Inventor: SAITO, Masahiro, Suita-shi Osaka 565-0871 (JP); MURAKAMI, Shinya, Suita-shi Osaka 565-0871 (JP); YONEDA, Toshiyuki, Suita-shi Osaka 565-0871 (JP); HASHIKAWA, Tomoko, Suita-shi Osaka 565-0871 (JP)
(74) Representative: Denison, Christopher Marcus
(86) International application number: PCT/JP2009/053096
(87) International publication number: WO 2010/021162

(57) **Abstract**

The present invention provides a cell preparation that can be prepared from cells collected from a patient of any age group, and that has an excellent bone tissue regenerative capacity and is effective for bone tissue regeneration. Undifferentiated osteoblasts obtained from alveolar bone, particularly those obtained by being cultured after the enzyme treatment of alveolar bone have a remarkable bone tissue regenerative capacity, and are therefore useful for a cell preparation for bone tissue regeneration.

## Description

### Technical Field

The present invention relates to a cell preparation having an excellent bone tissue regenerative capacity. The invention also relates to a cell group having an excellent bone tissue regenerative capacity, and a method of production thereof. The present invention also relates to a method for treating bone tissue damage with the cell preparation.

### Background Art

Techniques to repair or regenerate bone tissue damaged by factors such as disease, surgical operation, and physical impact have been vigorously studied in the field of dentistry and surgery. For the treatment of damaged bone tissue, it has been standard practice to artificially fix the damaged site of bone tissue, fill the damaged site with an autologous bone or a bone filler by transplantation, etc. A problem with these methods, however, is that the bone tissue may fail to recover to its normal state, or may take a long time to recover.

For example, in the field of dentistry, periodontal disease is a representative member of diseases associated with bone tissue damage. Periodontal disease is a lifestyle-related disease involving the loss of alveolar bone that anchors the teeth, affecting more than 50% of middle-aged and elderly individuals over the age of 40. In the advanced stage of periodontal disease, the teeth are lost, and patients impair the ability to bite. As a treatment for periodontal disease, techniques have been developed to regenerate lost periodontium using a Gore-Tex film or a filler produced from a pig tooth germ extraction fraction. However, while these techniques are effective for mild cases of periodontal disease, effective therapeutic effects cannot be expected for moderate to severe cases of periodontal disease.

In this connection, recently, techniques that repair or regenerate damaged bone tissue with a cell preparation capable of regenerating bone tissue have gained attention in the field of dentistry and surgery. For example, a method that uses the bone marrow stromal cells (BMSCs) of alveolar bone to regenerate bone tissue has been proposed (see, for example, Non-Patent Literature 1). However, with this technique, cells effective for the regeneration of bone tissue cannot be obtained from cells collected from middle-aged and elderly individuals, and the applicable area of bone tissue regenerative therapy using an autograft is limited by the age of a patient. Considering that periodontal disease has high morbidity in middle-aged and elderly individuals over the age of 60, it is considered clinically important to establish a technique that also enables preparation of cells effective for bone tissue regeneration from cells collected from middle-aged and elderly individuals.

Femur-derived osteoblasts that can regenerate bone tissue are commercially available. However, the femur-derived osteoblasts are not necessarily satisfactory in terms of bone tissue regenerative capacity, and development of cells that can more effectively regenerate bone tissue is demanded.

Considering the background of the prior art, there is a need to prepare cells having an excellent bone tissue regenerative capacity using cells collected from patients of any age group, and for the development of a cell preparation that can be used for bone tissue regeneration.

### Citation List

Non-Patent Literature
   NPL 1: Takehiro Mastubara et al., Alveolar Bone Marrow as a Cell Source for Regenera tive Medicine: Differences Between Alveolar and Iliac Bone Marrow Stromal Cells, Journal of Bone and Mineral Reseach, Vol. 20, No. 3, 2005, pages 399-409

### Summary of Invention

### Technical Problem

It is an object of the present invention to solve the problems of conventional techniques. Specifically, an object of the present invention is to provide a cell preparation that can be prepared from cells collected from patients of any age group, and that has an excellent bone tissue regenerative capacity and is effective for bone tissue regeneration.

### Solution to Problem

The present inventors conducted extensive studies to find a solution to the foregoing problems, and found that undifferentiated osteoblasts obtained from alveolar bone, particularly those obtained by being cultured after the enzyme treatment of alveolar bone have a remarkable bone tissue regenerative capacity, and are therefore highly useful for a cell preparation for bone tissue regeneration. It was also found that the cells also can be prepared from the alveolar bone of middle-aged and elderly individuals over the age of 60. The cells were found to be particularly effective for the regeneration of alveolar bone damaged by periodontal disease. The present invention has been completed based on these findings and upon further studies.

Specifically, the present invention provides the following aspects of the invention.
Item 1. A cell preparation for bone tissue regeneration, including alveolar bone-derived undifferentiated osteoblasts.
Item 2. A cell preparation according to Item 1, wherein the alveolar bone-derived undifferentiated osteoblasts are cells obtained by being cultured in a PDGF-containing medium after enzyme treatment of a collected alveolar bone tissue.
Item 3. A cell preparation according to Item 1, wherein the alveolar bone-derived undifferentiated osteoblasts are STMN2-, NEBL-, and MGP-expressing cells.
Item 4. A cell preparation according to Item 1, wherein the alveolar bone-derived undifferentiated osteoblasts are contained by being supported on a scaffold.
Item 5. A cell preparation according to Item 1, wherein the cell preparation is for alveolar bone regeneration.
Item 6. A method for producing a cell group that has a bone tissue regenerative capacity,
   the method including:
   a first step of subjecting a collected alveolar bone tissue to enzyme treatment to obtain alveolar bone-derived cells;
   a second step of culturing the alveolar bone-derived cells obtained in the first step in a medium that allows for growth of undifferentiated osteoblasts; and
   a third step of collecting a cell group grown in the second step.
Item 7. A production method according to Item 6, wherein the medium that allows for growth of undifferentiated osteoblasts used in the second step is a PDGF-containing medium.
Item 8. A production method according to Item 6, wherein the cell group collected in the third step is a group of cells expressing STMN2, NEBL, and MGP.
Item 9. A cell group that has a bone tissue regenerative capacity, and is obtained by culturing undifferentiated osteoblasts collected from alveolar bone.
Item 10. A cell group according to Item 9, wherein the undifferentiated osteoblasts collected from alveolar bone are cultured in a PDGF-containing medium.
Item 11. A cell group according to Item 9, wherein the undifferentiated osteoblasts are STMN2, NEBL, and MGP-expressing cells.
Item 12. A method for treating bone tissue damage,
   the method including the step of administering the cell preparation of Item 1 to a patient at a bone tissue site involving bone tissue damage.
Item 13. A use of alveolar bone-derived undifferentiated osteoblasts for manufacture of a cell preparation for bone tissue regeneration.
Item 14. A use according to Item 13, wherein the alveolar bone-derived undifferentiated osteoblasts are cells obtained by being cultured in a PDGF-containing medium after enzyme treatment of a collected alveolar bone tissue.
Item 15. A use according to Item 13, wherein the alveolar bone-derived undifferentiated osteoblasts are STMN2-, NEBL-, and MGP-expressing cells.

### Advantageous Effects of Invention

The present invention has succeeded, for the first time in the world, in developing a technique to prepare cells having a bone tissue regenerative capacity using cells obtained from middle-aged and elderly patients, and can thus provide a novel treatment by autograft for even middle-aged and elderly patients for which bone tissue regeneration treatment by autograft was not possible. Further, because the cell preparation of the present invention can be prepared from small amounts of alveolar bone tissue, it is less demanding for patients, and is highly useful in the clinic.

The cell preparation of the present invention is effective for the treatment of a variety of bone tissue damage, particularly for the regeneration of alveolar bone damaged by periodontal disease, and is particularly suited as a medicament for the treatment of periodontal disease, or as a medicament for alveolar bone regeneration. Further, because the cell preparation of the present invention uses cells that can even be prepared from the alveolar bone of middle-aged and elderly individuals, for whom morbidity from periodontal disease is high, the invention is also advantageous in providing an effective treatment for periodontal disease patients of any age group. Further, the cell preparation of the present invention is also useful in regenerative therapy performed after the removal of cancer tissue in metastatic bone, or after the treatment of osteosarcoma.

### Best Mode for Carrying Out the Invention

### 1. Cell Preparation for Bone Tissue Regeneration

A cell preparation of the present invention has use in bone tissue regeneration, and contains alveolar bone-derived undifferentiated osteoblasts. The cell preparation of the present invention is described below in detail.

In the present invention, "undifferentiated osteoblasts" means osteoblast cells yet to differentiate into osteoblasts that have a proliferative capacity and a potential to differentiate into osteoblasts. As used herein, "proliferative capacity" means the capacity of the undifferentiated osteoblasts to undergo cell division and proliferate in a medium that allows for growth of undifferentiated osteoblasts, as will be described later. Further, "potential to differentiate into osteoblasts" means the property to differentiate into osteoblasts either in the presence of an osteoblast inducing factor such as BMP2, or in the state of being supported on a scaffold. Differentiation into osteoblasts involves, for example, enhanced alkaliphosphatase activity, increased intensities of cell staining by alizarin red, or increased expression levels of osteoblast differentiation markers (RUNX2, OSTERIX, OSTEOCALCIN (OCN), BONE SIALOPROTEIN (BSP), OSTEOPONTIN (OPN)), and these can thus be used as an index of differentiation into osteoblasts.

The cells used for the cell preparation of the present invention are undifferentiated osteoblasts obtained from alveolar bone. Alveolar bone may be collected using common surgical procedures, or, more conveniently, the alveolar bone may be that which is removed during tooth extraction.

The undifferentiated osteoblasts used in the present invention can be obtained by first obtaining alveolar bone-derived cells by the enzyme treatment of alveolar bone, and then culturing the cells in a medium that allows for growth of undifferentiated osteoblasts.

The enzyme treatment of alveolar bone to obtain alveolar bone-derived cells can be performed by subjecting alveolar bone to an enzyme in a suitable buffer such as a phosphate buffer. The enzyme used to obtain alveolar bone-derived cells from alveolar bone may be an enzyme commonly used for the separation of cells from biological tissue fragments. Specific examples include proteases such as collagenase, pepsin, and trypsin. Of these enzymes, collagenase is preferred from the viewpoint of efficiently collecting cells from alveolar bone.

For improved efficiency of enzyme treatment, the collected alveolar bone may be fragmented into about 5 to 10 mm pieces prior to the enzyme treatment.

The conditions of the alveolar bone enzyme treatment are not particularly limited, as long as alveolar bone-derived cells can be liberated. For example, the enzyme treatment may be performed under the following conditions.
Alveolar bone concentration:
The alveolar bone concentration is set to generally about 3 alveolar bone pieces/ml, preferably about 1 alveolar bone piece/ml. Enzyme concentration:
   For example, when collagenase is used (> 1.5 U/mg), the enzyme concentration is set to generally about 1 to 4 mg/ml, preferably about 2 mg/ml. Note that, in this specification, 1 U of collagenase represents the enzyme titer that can liberate 1 micromole of 4-phenyl-azobenxyl-oxycarbonyl-L-prolyl-L-leucine from 4-phenyl-azobenxyl-oxycarbonyl-L-prolyl-L-leucyl-L-glycyl-L-proly l-D-arginine at 25°C in 1 min.
Process temperature:
About 37°C
Process time:
Generally, about 10 to 40 min, preferably about 20 min

The enzyme treatment of alveolar bone may be repeated multiple times as required, in order to increase the collection rate of alveolar bone-derived cells.

The enzyme treatment produces free alveolar bone-derived cells from the alveolar bone. The alveolar bone-derived cells can thus be obtained by treating these cells using a known technique such as centrifugation after the enzyme treatment.

A group of undifferentiated osteoblasts derived from alveolar bone can be obtained by culturing these alveolar bone-derived cells in a medium that allows for growth of undifferentiated osteoblasts.

The medium that allows for growth of undifferentiated osteoblasts may be a basal medium that contains essential components, such as inorganic salts, amino acids, and vitamins, for growing animal cells, supplemented with growth factors or other additional components required for the growth of undifferentiated osteoblasts. Examples of such basal medium include, for example, Eagle's minimum essential medium (MEM), α-Eagle's minimum essential medium (α-MEM), Dulbecco's modified Eagle's medium (DMEM), and Ham's F-12 medium.

Preferred examples of the medium that allows for growth of undifferentiated osteoblasts include media supplemented with the growth factor PDGF (platelet-derived growth factor). The presence of PDGF enables undifferentiated osteoblasts with an excellent bone tissue regenerative capacity to efficiently proliferate from the alveolar bone-derived cells. The PDGF added to the medium is classified into different forms, such as PDGFAA, PDGFAB, and PDGFBB, depending on the combination of subunits. The present invention can use any of such different forms. The origin of PDGF is not particularly limited, and, for example, PDGF may originate in animal tissue, or may be produced by genetic recombinant techniques. The concentration of PDGF added to medium may be generally 1 to 100 ng/ml, preferably 10 to 30 ng/ml.

Further, the medium that allows for growth of undifferentiated osteoblasts preferably includes monoethanolamine, in addition to PDGF. When monoethanolamine is added to the medium, the monoethanolamine concentration in the medium may be generally 0.1 to 100 µg/ml, preferably 6 µg/ml.

Further, the medium may be supplemented with one or more of insulin, transferrin, and bFGF (basic fibroblast growth factor). When insulin is added to the medium, the insulin concentration in the medium may be generally 1 to 100 µg/ml, preferably 10 µg/ml. When transferrin is added to the medium, the transferrin concentration in the medium may be generally 1 to 100 µg/ml, preferably 10 µg/ml. When bFGF is added to the medium, the bFGF concentration in the medium is generally 1 to 100 ng/ml, preferably 10 ng/ml.

Further, the medium that allows for growth of undifferentiated osteoblasts may contain antibiotics such as streptomycin, kanamycin, and penicillin.

Specifically, the preferred medium that allows for growth of undifferentiated osteoblasts is, for example, MF medium (Toyobo Co., Ltd.).

The alveolar bone-derived undifferentiated osteoblasts can be obtained by adding the alveolar bone-derived cells to the medium that allows for growth of undifferentiated osteoblasts, and culturing the cells at 37°C in 5% CO₂ for generally 1 to 7 days, preferably 4 days.

The cultured undifferentiated osteoblasts grown in this manner adhere to the bottom of the culture vessel, and thus the undifferentiated osteoblasts used for the cell preparation of the present invention can be obtained by collecting the cells adhering to the bottom of the culture vessel. The cells adhering to the bottom of the culture vessel can be collected according to known methods, for example, by subjecting the adhered cells at the bottom of the culture vessel to 0.25% trypsin and 1 mM EDTA (ethylenediaminetetraacetic acid).

The alveolar bone-derived undifferentiated osteoblasts used in the present invention have a far superior bone tissue regenerative capacity than conventionally known osteoblasts. Another notable feature of the undifferentiated osteoblasts is the long subculture period (for example, about 30 population doublings). Yet another feature of the undifferentiated osteoblasts is that the cells are induced to differentiate into bone cells in vitro in the presence of BMP-2 (bone morphogenetic protein-2). Note that, specifically, the undifferentiated osteoblasts can be differentiated into bone cells by culturing the cells in a medium that contains BMP-2 dexamethasone, ascorbic acid, and β-glycerophosphate. Still another feature of the undifferentiated osteoblasts is that the cells secrete BMP-2 upon being differentiated in the presence of BMP-2. These features represent evidence that the alveolar bone-derived undifferentiated osteoblasts differ from conventionally known osteoblasts.

The alveolar bone-derived undifferentiated osteoblasts used in the present invention also have a feature such that the cells express MGP (matrix gla protein: NM_000900 (National Center for Biotechnology (NCBI))), STMN2 (stathmin-like 2: NM_007029 (NCBI)), and NEBL (nebulette: NM_006393 (NCBI)). The expression levels of these genes are very low, or almost zero, in femur-derived osteoblasts. However, because these genes are expressed at high levels in the alveolar bone-derived undifferentiated osteoblasts used in the present invention, they can be used as novel markers specific to the alveolar bone-derived undifferentiated osteoblasts. More specifically, cells with expression levels of 70 or greater for MGP, 50 or greater for NEBL, and 50 or greater for STMN2 relative to the expression level 100 of β-Actin are specified as the alveolar bone-derived undifferentiated osteoblasts used in the present invention. The expression level of these genes can be measured using conventionally known methods, such as gene chip analysis, an RT-PCR method, and a real-time PCR method. The preferred measurement method of the gene expression level is gene chip analysis or a real-time PCR method. The gene chip used for the gene chip analysis may be a commercially available gene chip (for example, HT Human Genome U133 Array Plate Set (Gene Chip; Affymetrix, CA, U.S.A.)), or a gene chip produced according to a known method (Lipshutz, R. J. et al., (1999) Nature Genet. 21, Supplement, 20-24).

As required, the cell preparation of the present invention may include a pharmaceutically acceptable dilution carrier, in addition to the alveolar bone-derived undifferentiated osteoblasts. Examples of the pharmaceutically acceptable dilution carrier include physiological saline and buffer. The cell preparation of the present invention may further include a pharmacologically-active component as required.

Further, it is preferable that the cell preparation of the present invention contain alveolar bone-derived undifferentiated osteoblasts supported on a scaffold (platform). With the alveolar bone-derived undifferentiated osteoblasts supported on a scaffold, the graft survival rate of the alveolar bone-derived undifferentiated osteoblasts at the damaged site of bone tissue can be improved, and bone tissue regeneration can be further facilitated.

The scaffold used for the cell preparation of the present invention is not particularly limited, as long as it is pharmaceutically acceptable. For example, the scaffold may be a gel or porous, biodegradable or bioresorbable material. Preferred examples of usable scaffold include a fibrin gel (fibrin paste), hydroxyapatite, and a PGLA (poly DL-lactic-co-glycolic acid) -collagen sponge, of which fibrin gel is more preferable. The scaffold may be used alone, or in any combination of two or more.

The form of scaffold is not particularly limited, and may be appropriately designed according to the damaged site of bone tissue to which the cell preparation of the present invention is applied.

When the alveolar bone-derived undifferentiated osteoblasts are supported on a scaffold in the cell preparation of the present invention, the amount of alveolar bone-derived undifferentiated osteoblasts supported on a scaffold may be appropriately set according to such factors as the type of scaffold used. As an example, the alveolar bone-derived undifferentiated osteoblasts may be supported in an amount of generally about 3 × 10⁶ to 5 × 10⁶ cells, preferably about 4 × 10⁶ cells per 100 mg of the scaffold.

The technique used to support the cells on a scaffold is known, and the alveolar bone-derived undifferentiated osteoblasts can be supported on a scaffold using conventionally known techniques.

The cell preparation of the present invention has an excellent bone tissue regenerative capacity, and can be used to regenerate and restore the damaged bone tissue to normal conditions in diseases that involve bone tissue damage. The cell preparation of the present invention is applicable to any types of bone tissue damage such as in alveolar bone damaged by periodontal disease, bone damaged by osteosarcoma, bone damaged by metastatic cancer, and broken bone. However, from the viewpoint of exhibiting superior therapeutic (regenerative) effects, the cell preparation of the present invention is particularly suited to alveolar bone damaged by periodontal disease.

The cell preparation of the present invention is used by being administered (transplanted) to the damaged site of bone tissue. The method of administering (transplanting) the cell preparation of the present invention to the damaged site of bone tissue may be appropriately set according to factors such as the type of target bone tissue and the degree of damage.

The dose of the cell preparation of the present invention used in regenerating bone tissue with the cell preparation of the present invention may be appropriately set according to such factors as the degree of disease symptoms, and the sex and age of the patient. For example, the dose of the alveolar bone-derived undifferentiated osteoblasts for the damaged site of bone tissue may be set to about 2 × 10⁶ to 4 × 10⁶ cells, preferably about 3 × 10⁶ cells.

Note that the cell preparation of the present invention may be prepared as a cell preparation for autograft or allograft. However, from the viewpoint of suppressing rejection response, the cell preparation of the present invention is preferably prepared for autograft.

### 2. Cell Group Having Bone Tissue Regenerative Capacity, and Method of Production Thereof

The present invention also provides a cell group having a bone tissue regenerative capacity, and a method of production thereof. Specifically, the present invention provides a cell group having a bone tissue regenerative capacity that is obtained by culturing undifferentiated osteoblasts collected from alveolar bone. The present invention also provides a method for producing a cell group that has a bone tissue regenerative capacity, the method including the first step of obtaining alveolar bone-derived undifferentiated cells by enzyme treatment of collected alveolar bone tissue; the second step of culturing the alveolar bone-derived undifferentiated cells obtained in the first step in a medium that allows for growth of undifferentiated osteoblasts; and the third step of collecting a cell group (namely, a group of alveolar bone-derived undifferentiated osteoblasts) grown in the second step. The cell group and the producing method are as described in the foregoing section 1. Cell Preparation for Bone Tissue Regeneration.

### 3. Bone Tissue Damage Treatment Method

The present invention also provides a bone tissue damage treatment method that includes the step of administering the cell preparation for bone tissue regeneration to a patient at a bone tissue site involving bone tissue damage. Details of the treatment method, including the bone tissue damage to be treated, the cell preparation used, and the doses of cell preparation are as described in the foregoing section 1. Cell Preparation for Bone Tissue Regeneration.

### Examples

The present invention is described in detail below based on Examples. The present invention, however, is not limited by the following.

### Example 1: Preparation of Alveolar Bone-Derived Undifferentiated Osteoblasts

Alveolar bone-derived undifferentiated osteoblasts were prepared using the alveolar bone removed during the course of tooth extraction from four patients (age 66, 53, 52, and 27), who gave informed consent based on the resolution of the ethics committee. The alveolar bone was subjected to an enzyme reaction at 37°C for 20 min in 4-ml PBS (Phosphate buffered saline, pH 7.2) that contained 2 mg/ml of bacteria-derived collagenase (> 1.5 U/mg; Collagenase P, Roche). After the reaction, the bone was centrifuged with addition of an enzyme liquid and an equal amount of bovine serum to collect free cells, and the remaining alveolar bone was subjected again to enzyme treatment under the same conditions. This procedure was repeated, and a total of eight enzyme treatments were performed. Of the eight cell fractions collected after these enzyme treatments, the cell fractions collected after the first and second enzyme treatments were discarded, and the remaining six cell fractions were separately placed in a 35-mm culture plate that contained 4 ml of MF-start medium (Toyobo Co., Ltd., Tokyo, Japan), and cultured therein in 5% CO₂ at 37°C. Upon reaching 80% confluence in the culture plate, the cells were liberated with PBS supplemented with 0.25% trypsin/1 mM EDTA, and human alveolar bone-derived undifferentiated osteoblasts (HAOB) were collected. Note that the tests described below were conducted using HAOBs obtained from the cell fraction collected after the fifth enzyme treatment. Further, in the following, the alveolar bone-derived HAOBs obtained from the 66-, 53-, 52-, and 27-year old patients are denoted HAOB1, HAOB3, HAOB4, and HAOB5, respectively.

### Example 2: Proliferative Capacity Evaluation of Alveolar Bone-Derived Undifferentiated Osteoblasts

The HAOBs obtained in Example 1 were inoculated in MF medium (Toyobo Co., Ltd., Tokyo, Japan) at a concentration of 3 × 10⁴ cells/ml, and the cells were subcultured for 70 days with the medium replaced every 3 days. The population doubling (PD) of HAOBs during the course of cell growth was measured.

HAOB3 at PD 35 was treated with Colcemid (Karyo Max; Gibco BRL; 100 ng/ml for 6 h) and induced to enter interphase. The chromosome structure of the HAOB3 (about 50 cells) induced to enter interphase was then analyzed using a G-band method. The chromosome structure of the HAOB3 induced to enter interphase was also analyzed using a SKY (Spectral Karyotyping) method.

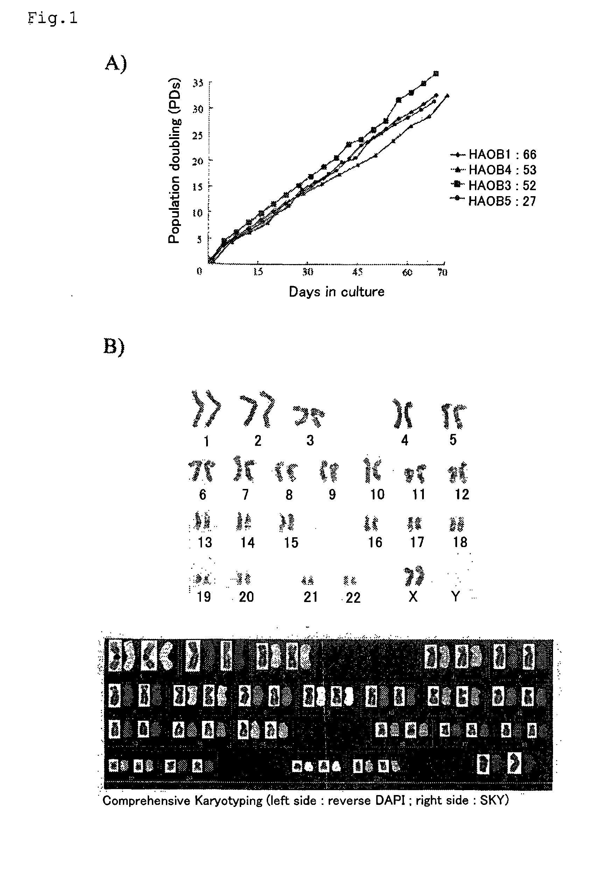

The results are presented in FIG. 1. It was confirmed from these results that the HAOBs (HAOB1 to 3) obtained from the alveolar bones of the middle-aged and elderly individuals had growth rates comparable to that of the HAOBs (HAOB4) obtained from the alveolar bone of the young adult. Further, from the normal diploid image observed in the chromosome structure analysis of the interphase HAOB3, the HAOBs were found to have a normal, stable proliferative capacity.

### Example 3: Proliferation Characteristic Evaluation of Alveolar Bone-Derived Undifferentiated Osteoblasts

The HAOB3 obtained in Example 1 was inoculated in a 96-well plate at a concentration of 2 × 10⁴ cells/well, and cultured in serum-free Dulbecco's modified Eagle's medium (DMEM) for 24 hours. The cells were further cultured for 96 hours in DMEM medium supplemented with 10 ng/ml bFGF, PDGFAA, PDGFAB, or PDGFBB, and cell proliferation activity was evaluated. As a control, the HAOB3 cultured in DMEM medium was cultured for 96 hours in serum-free or 20 volume% FCS-containing DMEM or in MF medium in the same manner, and cell proliferation activity was evaluated. Note that cell proliferation activity was measured using Celltiter-Glo luminescent cell viability assay (Promega) according to the manufacturer's protocol.

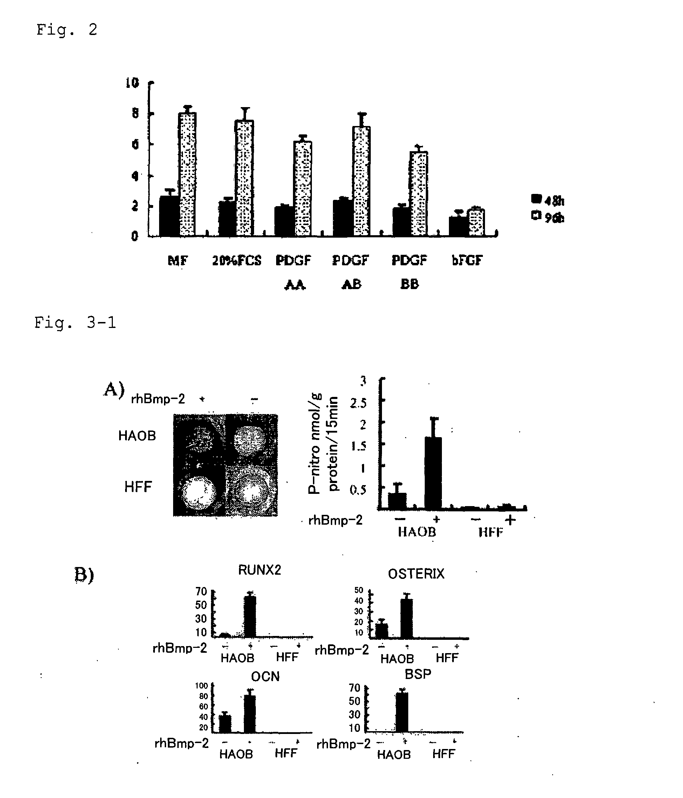

The results are presented in FIG. 2. The vertical axis in FIG. 2 represents the calculated value of cell proliferation activity relative to the value 1 assigned to the number of cells cultured in serum-free DMEM. It became clear from these results that HAOB proliferation significantly improves when PDGFAA, PDGFAB, or PDGFBB is added, and that the cell cultures grown in the presence of these growth factors are effective for the proliferation of HAOBs.

### Example 4: Evaluation of Alveolar Bone-Derived Undifferentiated Osteoblast Differentiation Potential (In vitro)-1

The HAOB3 (2 ml) obtained in Example 1 was inoculated in a culture plate at a concentration of 3 × 10⁴ cells/ml/cm², and the medium was replaced with 1 ml/cm² of MF medium (hereinafter, "rhBMP-2-added medium") supplemented with 100 nM dexamethasone, 50 µg/ml ascorbic acid, 10 mM β-glycerophosphate, and 100 ng/ml rhBMP-2 (recombinant human bone morphogenetic protein-2). The cells were then cultured for 9 days with the medium replaced every 3 days, and the differentiation characteristics of HAOB3 were evaluated. For comparison, the cells were cultured under the same conditions using a medium of the same composition as the rhBMP-2-added medium except that it did not contain rhBMP-2, and the differentiation characteristics of HAOB3 were evaluated.

Further, for comparison, the human foreskin fibroblast (HFF; Takara Bio Inc.) was cultured under the same conditions in rhBMP-2-added medium or in MF medium, and the differentiation characteristics of HFF were evaluated.

Note that, in this test, the differentiation characteristics were evaluated by alkaliphosphatase activity measurement, alizarin red staining, measurement of the expression levels of osteoblast differentiation markers (RUNX2, OSTERIX, OSTEOCALCIN (OCN), BONE SIALOPROTEIN (BSP)), and immunostaining using antibodies against OSTEOPONTIN (OPN) and OCN. The specific measurement conditions are as follows.

### <Alkaliphosphatase Activity Measurement>

After fixing the cells with 4% paraformaldehyde for 20 min, alkaliphosphatase activity was measured by performing a reaction at room temperature in a 0.1 M TRIS-HCl (pH 8.5) solution that contained 0.1 mg/ml naphthol AS-MX phosphate (Sigma), 0.5% N-N dimethyl formamide (Sigma), 2 mM MgCl₂, and 0. 6 mg/ml Fast Blue BB salt (Sigma) .

### <Alizarin Red Staining>

The cells were detected by 2% alizarin red S (pH 6.4; Sigma) staining after fixing the cells with 4% paraformaldehyde for 20 min.

### <Measurement of Expression Levels of Osteoblast Differentiation Markers>

Total RNA was extracted from the cells, and the expression levels of RUNX2, OSTERIX, OCN, and BSP were measured using a PCRmethod. The extraction of total RNA from the cells was performed using Isogen (Nippon Gene, Tokyo, Japan) according to the manufacturer's protocol. cDNA was prepared using 1 µg of total RNA and reverse transcriptase (M-MLV reverse transcriptase, Invitrogen Corporation). The mRNA expression level was analyzed with Power SYBR Green PCR Master Mix (Applied Biosystems), using an AB 7300 Real-Time PCR System (Applied Biosystems). The sequences of the primers used are as follows.
OSTERIX (Forward primer: CTGAAGAATGGGTGGGGAAGG (SEQ ID NO: 1), reverse primer: GGCCTCTGTCCTCCTAGCTC (SEQ ID NO: 2))
RUNX2 (Forward primer: GAAACTCAACAGATTAACTATCGTTTGC (SEQ ID NO: 3), Reverse primer: GAATTTATCACAGATGGTCCCTAATGG (SEQ ID NO: 4))
OSTEOCALCIN (Forward primer: CACACTCCTCGCCCTATTGG (SEQ ID NO: 5), Reverse primer: TGCACCTTTGCTGGACTCTG (SEQ ID NO: 6))
BONE SIALOPROTEIN (Forward primer: CGAATACACGGGCGTCAATG (SEQ ID NO: 7), Reverse primer: GTAGCTGTACTCATCTTCATAGGC (SEQ ID NO: 8))
BMP2 (Forward primer: CCAGAAACGAGTGGGAAAAC (SEQ ID NO: 9), Reverse primer: AATTCGGTGATGGAAACTGC (SEQ ID NO: 10))
Glyceraldehyde-3-phosphate dehydrogenase (GAPDH) (Forward primer: AAGAGCACAAGAGGAAGAGAGAGAC (SEQ ID NO: 11), Reverse primer: TTATTGATGGTACATGACAAGGTG (SEQ ID NO: 12)).

### <Immunostaining using Antibodies against OPN and OCN>

The cells were fixed with 4% paraformaldehyde, and the expression levels of OPN and OCN were measured by immunostaining using antibodies against OPN and antibodies against OCN.

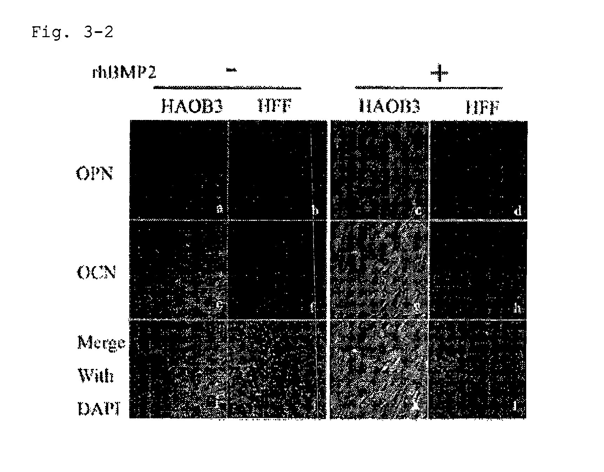

The results are presented in FIG. 3-1 and FIG. 3-2. The result of alizarin red staining is shown in FIG. 3-1 A) (left), and the result of alkaliphosphatase activity measurement is shown on the right. As shown on the left in FIG. 3-1 A), the HAOB3 cultured in rhBMP-2-added medium was strongly stained with alizarin red, expressing high calcification capacity. Further, as shown on the right in FIG. 3-1 A), the HAOB3 cultured in rhBMP-2-added medium showed a significant improvement in alkaliphosphatase activity measured as an index of bone formation. In contrast, HFF was not stained with alizarin red even after being cultured in rhBMP-2-added medium, and did not show alkaliphosphatase activity.

FIG. 3-1 B) presents the measurement results of RUNX2, OSTERIX, OCN, and BSP expression levels. The expression level of each differentiation marker shown in FIG. 3-1 B) is a relative value with respect to the expression level 100 of β-Actin. It can be seen from these results that the RUNX2, OSTERIX, OCN, and BSP expression levels increase in the HAOB3 cultured in rhBMP-2-added medium, and expression of bone-forming capacity was confirmed. In contrast, HFF did not show expression of the differentiation markers even after being cultured in rhBMP-2-added medium or in MF medium.

FIG. 3-2 represents the results of immunostaining performed with antibodies against OPN and OCN. As can be seen in FIG. 3-2, in contrast to the low OPN and OCN expression levels in the HAOB3 not stimulated with rhBMP-2, a strong, positive response to the OPN and OCN antibodies was observed in the rhBMP-2-stimulated HAOB3. The results thus confirmed that the HAOBs have the properties of undifferentiated osteoblasts before being induced to differentiate, and differentiate into OPN-positive and OCN-positive osteoblasts upon rhBMP-2 stimulation.

### Example 5: Evaluation of Alveolar Bone-Derived Undifferentiated Osteoblast Differentiation Potential (In vitro)-2

The HAOB3 obtained in Example 1 was inoculated in MF medium (Toyobo Co., Ltd., Tokyo, Japan) at a concentration of 3 × 10⁴ cells/ml, and cultured therein until PD 29 with the medium replaced every 3 days. The HAOB3 at PD 6, 11, 16, 21, 24, and 29 was cultured in rhBMP-2-added medium under the conditions of Example 4, and evaluated by alizarin red staining, and by the measurement of the osteoblast differentiation marker (RUNX2, OSTERIX, OCN, BSP) expression levels.

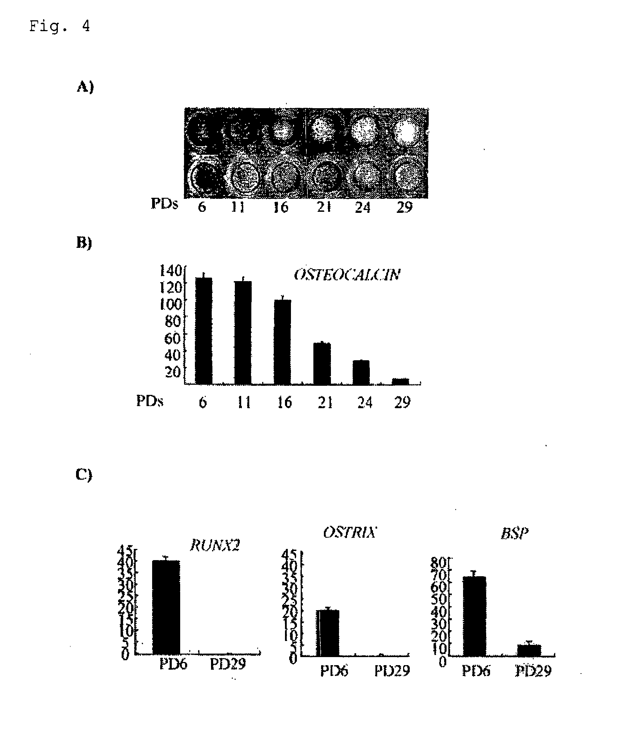

The results are presented in FIG. 4. FIG. 4 A) shows the results of alizarin red staining, FIG. 4 B) shows the results of OCN expression level measurement, and FIG. 4 C) shows the results of RUNX2, OSTERIX, and BSP expression level measurement. The expression level of each differentiation marker presented in FIG. 4 B) and C) is a relative value with respect to the expression level 100 of β-action. As clearly shown in FIG. 4, even the HAOB3 at PD 16 was able to express a sufficient level of bone-forming capacity, and had an osteoblast differentiation potential.

### Example 6: Evaluation of Differentiation Potential of Alveolar Bone-Derived Undifferentiated Osteoblasts into Bone Cells (In vitro)-3

The HAOB3 obtained in Example 1 was inoculated in a culture plate at a concentration of 3 × 10⁴ cells/ml/cm², and the medium was replaced with an MF medium that contained 100 nM dexamethasone, 50 µg/ml ascorbic acid, 10 mM β-glycerophosphate, and 50, 100, 200, 500, or 1,000 ng/ml rhBMP-2. The cells were cultured for 9 days with the medium replaced every 3 days, and the differentiation characteristics of HAOB3 were evaluated. The differentiation characteristics were evaluated in the same manner as in Example 4, by alkaliphosphatase activity measurement, alizarin red staining, and the measurement of osteoblast differentiation marker (RUNX2, OSTERIX, OCN, BSP) expression levels.

For comparison, femur-derived osteoblasts (NHOst; Lonza Walkersville Inc., MD, USA) were cultured under the same conditions, and differentiation characteristics were evaluated in the same manner.

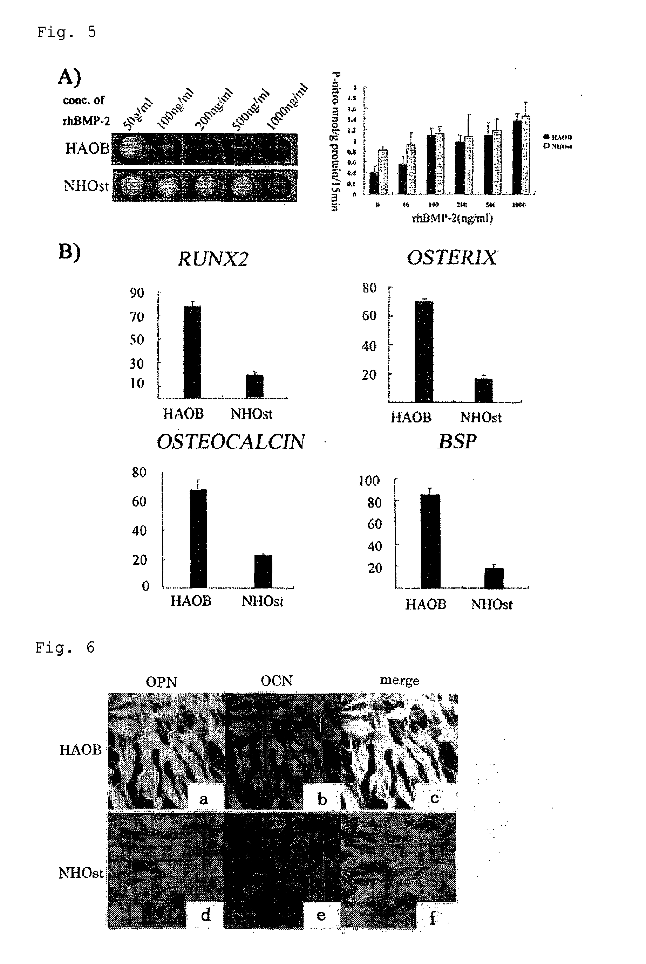

The results are presented in FIG. 5. FIG. 5 A) shows the results of alizarin red staining (left), and the results of alkaliphosphatase activity measurement (right). As can be seen in these results, while HAOB3 and NHOst had substantially the same levels of alkaliphosphatase activity, stronger expression of bone-forming and calcification capacity was confirmed in HAOB3 through formation of an alizarin red-staining positive calcified product at low rhBMP-2 concentrations.

FIG. 5 B) represents the measurement results of the RUNX2, OSTERIX, and BSP expression levels in HAOB3 and NHOst induced to differentiate with 100 ng/ml of rhBMP-2. The expression level of each differentiation marker shown in FIG. 5 B) is a relative value with respect to the expression level 100 of β-Actin. It can be seen from these results that HAOB3 is induced to differentiate more strongly than NHOst at 100 ng/ml rhBMP-2, confirming stronger expression of bone-forming capacity.

### Example 7: Evaluation of Alveolar Bone-Derived Undifferentiated Osteoblast Differentiation Potential (In vitro)-4

HAOB3 or NHOst was mixed with an MF medium that contained 100 nM dexamethasone, 50 µg/ml ascorbic acid, 10 mM β-glycerophosphate, and 100 ng/ml rhBMP-2 (3 × 10⁴ cells/ml/cm²), and 0.5 ml of the mixture was added onto a glass coverslip. The cells were then cultured in 5% CO₂ at 37°C for 72 hours. After removing the culture supernatant from the glass coverslip, the cells were fixed with a 50% acetone/methanol solution for 2 min, and blocked with PBS that contained 1 mg/ml of bovine albumin. The blocked cells on the glass coverslip were then acted upon by anti-OCN monoclonal antibody (clone GluOC4-5, Takara, Tokyo, Japan), and allowed to react with anti-mouse alexa 488 secondary antibody (Invitrogen Corporation) for OCN staining. Further, the blocked cells on the glass coverslip were acted upon by anti-OSTEOPONTIN (OPN) polyclonal antibody (O-17, IBL, Gunma, Japan), and allowed to react with anti-rabbit alexa 555 secondary antibody (Invitrogen Corporation) for OPN staining. The nuclear staining against the blocked cells on the glass coverslip was performed with DAPI (4',6-diamino-2-phenylindole).

The stained cells were observed with a confocal laser microscope (LSM510; Carl Zeiss MicroImaging, Jena, Germany) using 403-, 488-, and 543-nm laser beams. The results are presented in FIG. 6. FIG. 6 represents the OPN-stained HAOB3 after differentiation (a), the OCN-stained HAOB3 after differentiation (b), an overlaid image of a and b (c), the OPN-stained NHOst after differentiation (d), the OCN-stained NHOst after differentiation (e), and an overlaid image of d and e (f). It can be seen from these results that the differentiated HAOB3 cells all show a positive response to anti-OPN antibody and anti-OCN antibody, and have OPN and OCN expression levels considerably higher than those of NHOst.

### Example 8: Evaluation of Alveolar Bone-Derived Undifferentiated Osteoblast Differentiation Characteristics

The HAOB3 or NHOst obtained in Example 1 was inoculated in a culture plate at a concentration of 3 × 10⁴ cells/ml/cm², and the medium was replaced with 1 ml/cm² of MF medium that contained 100 nM dexamethasone, 50 µg/ml ascorbic acid, 10 mM β-glycerophosphate, and 100 ng/ml of rhBMP-2. The cells were cultured for 9 days with the medium replaced every 3 days. The expression level of BMP-2 was then measured for the cells cultured for 9 days, using a real-time PCR method. Note that the collection of RNA from the cells and cDNA synthesis were performed using the techniques described in Example 4. RT-PCR was performed using Takara EX Taq (Takara, Tokyo, Japan) according to the manufacturer's protocol.

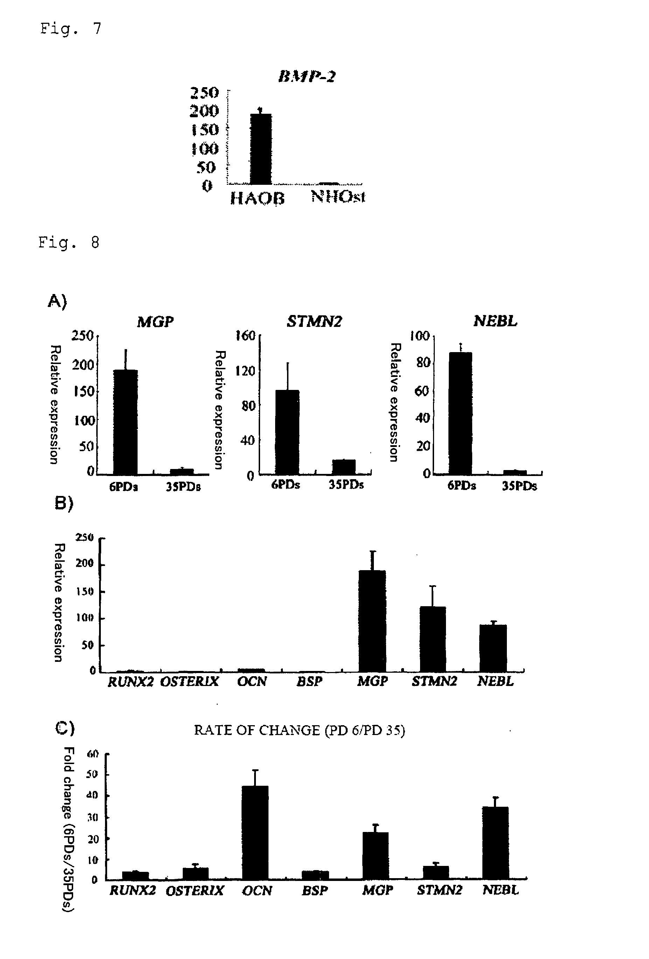

The results are presented in FIG. 7. As shown in FIG. 7, the cells differentiated from HAOB3 had a BMP-2 expression level 200 times or higher than that in cells differentiated from NHOst. The result therefore demonstrated that HAOB3 had characteristics that were clearly different from the characteristics of NHOst.

### Example 9: Analysis of Genes Specific to Alveolar Bone-Derived Undifferentiated Osteoblasts

The expression of genes specific to alveolar bone-derived undifferentiated osteoblasts was analyzed by conducting a test, as follows.

The HAOB1 to 4 obtained in Example 1 were inoculated in MF medium (Toyobo Co., Ltd., Tokyo, Japan) at a concentration of 3 × 10⁴ cells/ml, and cultured therein until PD 35 with the medium replaced every 3 days.

Then, total RNA was extracted from HAOB at PD 6, 11, 16, 21, 24, 29, and 35, and the strength of gene expression in HAOB at PD 6 and 35 was analyzed. The gene expression strength was analyzed using an HT Human Genome U133 Array Plate Set (Gene Chip; Affymetrix, CA, U.S.A.) that contained full-length probes for about 33,000 genes, according to the Affymetrix manual [http://www.affymetrix.com/support/technical/index.affx]. Data analysis was performed using Gene Chip Operation System (Affymetrix CA, U.S.A.) and GeneSpringGX software (Silicon Genetics). Changes in staining levels among the chips were normalized using the average of the median values of all measured values from the genes on the chip. Further, eigenvectors were created in the raw data and logarithmically converted data, using a principal component analysis (PCA) method that uses singular value decomposition (SVD) (reference: Kami D, Shiojima I, Makino H, Matsumoto K, Takahashi Y, Ishii R, Naito AT, Toyoda M, Saito H, Watanabe M, Komuro I, Umezawa A: *Gremlin* e*nhances the determined path to cardiomyogenesis*, PLoS ONE, 3:e2407.2008).

Further, for comparison, gene expression strength was also analyzed in the same manner for human foreskin fibroblasts (HFF; Takara Bio), human osteosarcoma cells (MG63; Riken BioResource Center), and femur-derived osteoblasts (NHOst; Lonza Walkersville Inc., MD, USA).

The PCA analysis found the existence of a gene group whose expression levels lowered with an increase in PD. These genes were screened for genes that were particularly expressed at a high level in HAOBs, and the gene expression level was measured using real-time PCR. The results are presented in FIG. 8 A). The expression level of each gene in FIG. 8 A) is a relative value with respect to the expression level 100 of β-action. As shown in FIG. 8 A), while STMN2, NEBL, and MGP were expressed at high levels in HAOBs at PD 6, these genes were hardly expressed in HAOB3 at PD 35. The expression of these genes and the expression levels of the bone marker genes RUNX2, OSTERIX, OSTEOCALCIN, and BSP in HAOB3 at PD 6 were measured using real-time PCR. The results are presented in FIG. 8 B). The expression level of each gene in FIG. 8 B) is a relative value with respect to the expression level 100 of β-Actin. As shown in FIG. 8 B), STMN2, NEBL, and MGP clearly had high expression levels. Further, as shown in FIG. 8 C), OCN had the greatest rate of change in the analysis of gene change rate in HAOB3 at PD 6 and 35. However, because the expression level of OCN is low in cells at PD 6, OCN is considered unsuitable as an identification marker for HAOBs.

The expression levels of STMN2, NEBL, and MGP in human foreskin fibroblasts (HFF), human osteosarcoma cells (MG63), femur-derived osteoblasts (NHOst), and HAOB1 to 4 at PD 6 were also measured using real-time PCR. The sequences of the primers used in the real-time PCR are as follows.
STMN2 (Forward primer: acgtctgcaggaaaaggaga (SEQ ID NO: 13), Reverse primer: acgatgtagtcgccgtctct (SEQ ID NO: 14))
NEBL (Forward primer: cattcccaaggctatggcta (SEQ ID NO: 15), Reverse primer: acgatgtagtcgccgtctct (SEQ 10 NO: 16))
MGP (Forward primer: cgcttcctgaagtagcgatt (SEQ ID NO: 17), Reverse primer: ccctcagcagagatggagag (SEQ ID NO: 18))

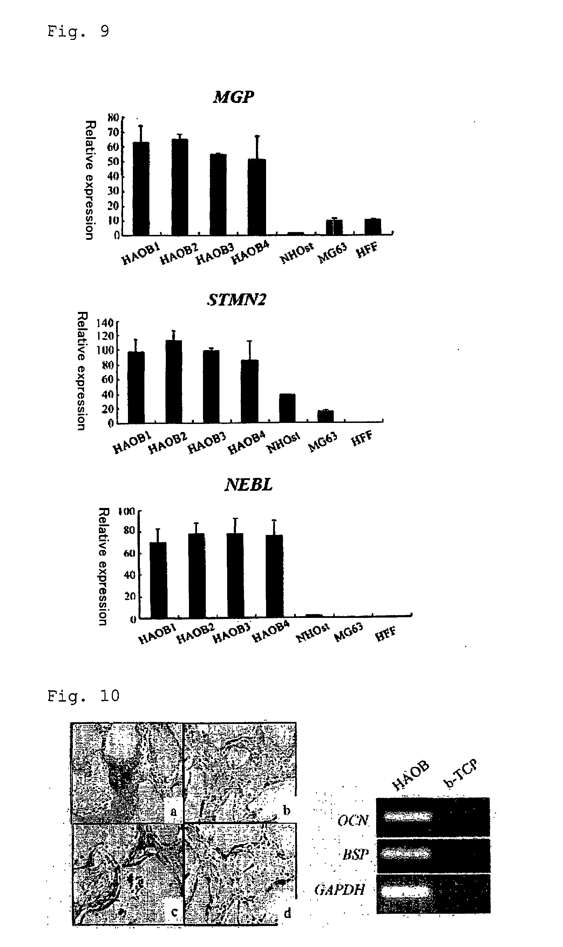

The results are presented in FIG. 9. The expression level of each gene shown in FIG. 9 is a relative value with respect to the expression level 100 of β-Actin. As can be seen in FIG. 9, HAOBs clearly had higher STMN2, NEBL, and MGP expression levels.

These results confirmed that the expression of STMN2, NEBL, and MGP could indeed be used as a specific marker for HAOBs.

### Example 10: Evaluation of Bone-Forming Capacity of Alveolar Bone-Derived Undifferentiated Osteoblasts in Vivo

HAOBs were examined regarding their differentiation potential in vivo by conducting a transplantation experiment using CB-17 scid/scid (SCID) mice (Nihoncrea, Tokyo, Japan) according to the method of Handa et al. (K. Handa et al., 2002, Cementum matrix formation in vivo by cultured dental follicle cells. Bone. 31(5):606-611.).

First, 1.5 × 10⁶ HAOB3 cells were mixed with 40 mg of hydroxyapatite powder (Osferion, Olympus, Tokyo, Japan) in 1 ml MF medium. The cells were cultured for 12 hours, mixed with 30 µl of a fibrin paste (a mixture of mouse fibrinogen (15 µl) and thrombin (15 µl); Sigma), and subcutaneously transplanted into the back of an SCID mouse, 5 weeks of age. After 4 weeks, a graft (hereinafter, "HAOB3-administered graft") was removed. A half of the removed graft was sent for expression analysis of bone marker genes (OCN, BSP, GAPDH), and the remaining half was sent for histochemical analysis.

For comparison, NHOst was used instead of HAOB3 and subcutaneously transplanted into the back of an SCID mouse under the same conditions, and a graft (hereinafter, "NHOst-administered graft") removed 4 weeks after the transplantation was analyzed in the same manner.

Further, for comparison, a mixture of hydroxyapatite powder and fibrin paste without cells was subcutaneously transplanted into the back of an SCID mouse under the same conditions, and a graft (hereinafter, "cell-free graft") removed 4 weeks after the transplantation was tested in the same manner.

Note that the marker gene expression analysis and histochemical analysis of the grafts were performed according to the following methods.

### <Graft Marker Gene Expression Analysis>

The collection of RNA from the grafts, and cDNA synthesis were performed using the techniques described in Example 4. For the measurement of OCN, BSP, and GAPDH mRNA expression levels, RT-PCR was performed using Takara EX Taq (Takara, Tokyo, Japan) according to the manufacturer's protocol.

### <Graft Histochemical Analysis>

The grafts were fixed with 4% paraformaldehyde for 24 hours, decalcified with 10% formic acid for 3 days, and embedded in paraffin to prepare 4-µm sections.

The morphology of the paraffin-embedded sections was then observed by hematoxylin-eosin staining.

Further, the paraffin-embedded sections were immunostained using human-specific vimentin antibodies. Immunostaining was performed according to the method of Handa et al. (K. Handa et al., 2002, Cementum matrix formation in vivo by cultured dental follicle cells. Bone. 31(5):606-611.), using an M.O.M kit (Vector Burlingame). Specifically, anti-vimentin monoclonal antibodies (V9, Dako, Carpinteria, CA, USA) diluted with PBS containing 1 mg/ml of bovine albumin were allowed to react with the paraffin-embedded sections for 1 hour, and then with biotinylated secondary antibodies and peroxidase-binding avidin for color reaction with diaminobenzidine. Note that normal mouse IgG was used as a control in the immunostaining.

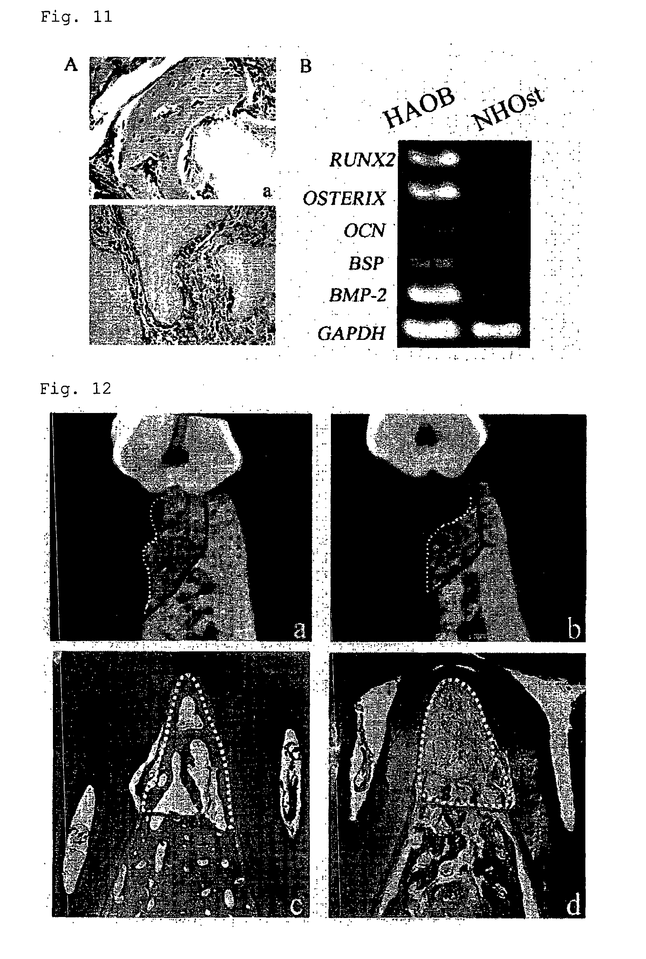

The results are presented in FIGS. 10 and 11. In FIG. 10, the results of graft histochemical analysis are shown on the left. FIG. 10 represents a HAOB3-administered graft after hematoxylin-eosin staining (a); a HAOB3-administered graft immunostained with human-specific vimentin antibodies (c); a cell-free graft after hematoxylin-eosin staining (b); and a cell-free graft immunostained with human-specific vimentin antibodies (d). In the HAOB3-administered graft, the bone cells in the bone-forming structure were immunostained with the human-specific vimentin antibodies, confirming the presence of HAOB3-differentiated bone cells in the tissue fragment. In contrast, the cell-free graft was not immunostained with human specific vimentin antibodies.

In FIG. 10, the measurement results of OCN, BSP, and GAPDH mRNA expression level in the cells contained in the HAOB3-administered graft are shown on the right. The results show strong expression of OCN, BSP, and GAPDH in the HAOB3-administered graft, demonstrating differentiation of HAOB3 into the bone cells.

FIG. 11 presents the results of the comparison between the HAOB3-administered graft, NHOst-administered graft, and cell-free graft. FIG. 11 A) shows a HAOB3-administered graft after hematoxylin-eosin staining (a), and a NHOst-administered graft after hematoxylin-eosin staining (b). In contrast to the HAOB3-administered graft in which the formation of a bone-forming structure was observed, bone formation was not sufficient in the NHOst-administered graft.

FIG. 11 B) presents the measurement results of RUNX2, OSTERIX, OCN, BSP, and BMP-2 expression levels in the HAOB3-administered graft, NHOst-administered graft, and cell-free graft. The results confirmed higher expression levels of the bone marker genes (RUNX2, OSTERIX, OCN, and BSP) and BMP-2 in the HAOB3-administered graft than in the NHOst-administered graft.

Specifically, the results of marker gene expression and histochemical analyses provided evidence that HAOB3 has a far superior bone-forming capacity in vivo compared to the commercially available femur-derived osteoblast NHOst.

### Example 11: Evaluation of Alveolar Bone Regenerative Capacity of Alveolar Bone-Derived Undifferentiated Osteoblasts in Vivo 1. Creation of Periodontal Disease Animal Model

A female beagle, 2.5 years of age, was used in the test. The gum at the left and right mandibular fourth premolar and first last molar regions was detached under general anesthesia, and a cleaved bone defect (3 × 5 mm; class II furcation defect) was created at the furcation area of these premolars and last molars. Silicone putty was then inserted into the defect areas. After 1 month, the silicone putty was removed, and formation of a furcation defect was confirmed. Root planing was then performed on the root surface, and a graft bed was created to produce a periodontal disease dog model.

### 2. Preparation of Dog Alveolar Bone-Derived Undifferentiated Osteoblasts

An about 3 to 5 mm alveolar bone fragment surgically removed during the creation of the periodontal disease animal model was placed in 4 ml of PBS (Phosphate buffered saline, pH 7.2) that contained 2 mg/ml of bacteria-derived collagenase (> 1.5 U/mg; Collagenase P, Roche), and subjected to enzyme reaction at 37°C for 20 min. After the reaction, a bovine serum was added in an amount equal to that of the enzyme reaction liquid, and the cells liberated by centrifugation were collected. The remaining alveolar bone was subjected to enzyme treatment again under the same conditions. This procedure was repeated, and a total of five enzyme treatments were performed. The free cells after the fifth enzyme treatment were collected, and cultured in 5% CO₂ at 37°C in a 35-mm culture plate that contained 2 ml of MF-start medium (Toyobo Co., Ltd., Tokyo, Japan). Upon reaching 80% confluence in the culture plate, the cells were liberated with PBS that contained 0.25% trypsin/1 mM EDTA, and dog alveolar bone-derived undifferentiated osteoblasts (DAOBs) were collected.

The DAOBs (1.5 × 10⁶ cells) were supported on a 30-µl fibrin gel to prepare a DAOB-containing cell preparation.

### 3. DAOB Transplantation in Periodontal Disease Animal Model, and Results

The DAOB-containing cell preparation was autografted to the bone defect site of the periodontal disease dog model (500,000 DAOB cells/bone defect site). As a control, a fibrin gel was transplanted alone without the DAOB-containing cell preparation.

Jaw bone was removed 6 weeks after transplantation, and the amount of bone regeneration was determined by micro-CT. After trimming the transplanted site, the specimen was decalcified for 2 months with formic acid, and a paraffin specimen was prepared and then stained with hematoxylin-eosin for microscope observation.

The results are shown in FIG. 12. FIG. 12 represents the result of the micro-CT analysis of the affected site that had the DAOB-containing cell preparation transplant (a), the result of the micro-CT analysis of the affected site that had only the fibrin gel transplant (b), the hematoxylin-eosin stained affected site that had the DAOB-containing cell preparation transplant (c), and the hematoxylin-eosin stained affected site that had only the fibrin gel transplant (d).

As clearly shown in FIG. 12, new bone formation was observed in the bone defect site that had the DAOB-containing cell preparation transplant (FIG. 12, a), and histologically notable formation of new bone was confirmed (FIG. 12, c). In contrast, sufficient formation of new bone was not observed in the bone defect site that had only the fibrin gel transplant (FIG. 12, b and d).

These results demonstrated new bone formation and bone tissue regeneration by the alveolar bone-derived undifferentiated osteoblasts by actual animal experimentation. Specifically, the test results provide evidence that the cell preparation of the present invention has potential in clinical use, and is useful for curing bone tissue damage through bone tissue regeneration.

### Brief Description of Drawings

FIG. 1 A) represents the results of PD measurement using HAOBs obtained from the alveolar bone of different age groups; FIG. 1 B) represents the results of the karyotyping of HAOB3 (PD 35) induced to enter interphase, performed by using the G-band method (top) and the SKY method (bottom).
FIG. 2 represents the evaluation results of the cell proliferation activity of HAOB3 cultured in media supplemented with various growth factors; the vertical axis represents the calculated value of cell proliferation activity relative to the value 1 assigned to the number of cultured cells in serum-free DMEM; MF, MF medium; 20% FCS, 20 volume% FCS-containing DMEM medium; PDGFAA, DMDM medium supplemented with 10 ng/ml PDGFAA; PDGFAB, DMEM medium supplemented with 10 ng/ml PDGFAB; PDGFBB, DMEM medium supplemented with 10 ng/ml PDGFBB; bFGF, DMEM medium supplemented with 10 ng/ml bFGF.
FIG. 3-1 A) represents the results of the alizarin red staining of HAOB3 cultured in rhBMP-2-added medium or MF medium (left), and the measurement results of alkaliphosphatase activity (right); FIG. 3-1 B) represents the measurement results of RUNX2, OSTERIX, OCN, and BSP expression levels in HAOB3 cultured in rhBMP-2-added medium or MF medium; the vertical axis in B) represents the calculated value of the expression level of each differentiation marker relative to the expression level 100 of β-Actin.
FIG. 3-2 represents HAOB3 cultured in rhBMP-2-added medium or MF medium and stained with antibodies against OPN and OCN.
FIG. 4 A) represents HAOB3 at PD 6, 11, and 16 cultured in rhBMP-2-added medium or MF medium and stained with alizarin red; FIG. 4 B) represents the measurement results of OCN expression level in HAOB3 at PD 6, 11, and 16 cultured in rhBMP-2-added medium; FIG. 4C) represents the measurement results of RUNX2, OSTERIX, and BSP expression levels in HAOB3 at PD 6 cultured in rhBMP-2-added medium; the vertical axis in B) and C) represents the calculated value of the expression level of each differentiation marker relative to the expression level 100 of β-Actin.
FIG. 5 A) represents the results of the alizarin red staining of HAOB3 and NHOst cultured in medium containing rhBMP-2 at a concentration of 50 to 1000 ng/ml (left), and the measurement results of alkaliphosphatase activity (right); FIG. 5 B) represents the measurement results of RUNX2, OSTERIX, OCN, and BSP expression levels in HAOB3 and NHOst cultured in medium containing rhBMP-2 at a concentration of 100 ng/ml; the vertical axis in B) represents the calculated value of the expression level of each differentiation marker relative to the expression level 100 of β-action.
FIG. 6 represents the OPN-stained HAOB3 after differentiation (a), the OCN-stained HAOB3 after differentiation (b), an overlaid image of a and b (c), the OPN-stained NHOst after differentiation (d), the OCN-stained NHOst after differentiation (e), and an overlaid image of d and e (f).
FIG. 7 represents the measurement results of BMP-2 expression levels in HAOB3 and NHOst cultured in medium containing hBMP-2 at a concentration of 100 ng/ml; the vertical axis represents the calculated value of hBMP-expression level relative to the expression level 100 of β-Actin.
FIG. 8 A) represents the measurement results of MGP, STMN2, and NEBL expression levels in HAOB3 (PD 6 and 35); the vertical axis in A) represents the calculated value of the expression level of each gene relative to the expression level 100 of β-Actin; FIG. 8 B) represents the measurement results of RUNX2, OSTERIX, OCN, BSP, MGP, STMN2, and NEBL expression levels in HAOB3 (PD 6) ; the vertical axis in B) represents the calculated value of the expression level of each gene relative to the expression level 100 of β-Actin; FIG. 8 C) represents the rate of change of RUNX2, OSTERIX, OCN, BSP, MGP, STMN2, and NEBL gene expression (%) in HAOB3 at PD 6 and 35; the rate of change of gene expression is calculated by (gene expression level in HAOB3 at PD 6/gene expression level in HAOB3 at PD 35) × 100.
FIG. 9 represents the measurement results of STMN2, NEBL, and MGP expression levels in HAOB1 to 4, human foreskin fibroblasts (HFF), human osteosarcoma cells (MG63), and femur-derived osteoblasts (NHOst); the vertical axis in each diagram represents the calculated value of the expression level of each gene relative to the expression level 100 of β-Actin.
FIG. 10 on the left shows a HAOB3-administered graft after hematoxylin-eosin staining (a), a HAOB3-administered graft immunostained with human specific vimentin antibodies (c), a HAOB3-free graft after hematoxylin-eosin staining (b), and a HAOB3-free graft immunostained with human specific vimentin antibodies (d); FIG. 10 on the right represents the measurement results of OCN, BSP, and GAPDH mRNA expression levels in a HAOB3-administered graft; the lane under b-TCP represents the measurement result of the expression level of each mRNA in an experiment conducted with only hydroxyapatite, without mixing the cells.
FIG. 11 A) shows a HAOB3-administered graft after hematoxylin-eosin staining (a), a NHOst-administered graft after hematoxylin-eosin staining (b); FIG. 11 B) represents the measurement results of RUNX2, OSTERIX, OCN, BSP, and BMP-2 expression levels in a HAOB3-administered graft and a NHOst-administered graft.
FIG. 12 represents the result of the micro-CT analysis of the affected site that had the DAOB-containing cell preparation transplant (a), the result of the micro-CT analysis of the affected site that had only the fibrin gel transplant (b), the hematoxylin-eosin stained affected site that had the DAOB-containing cell preparation transplant (c), and the hematoxylin-eosin stained affected site that had only the fibrin gel transplant (d).

## Claims

1. A cell preparation for bone tissue regeneration, comprising alveolar bone-derived undifferentiated osteoblasts.

2. A cell preparation according to claim 1, wherein the alveolar bone-derived undifferentiated osteoblasts are cells obtained by being cultured in a PDGF-containing medium after enzyme treatment of a collected alveolar bone tissue.

3. A cell preparation according to claim 1, wherein the alveolar bone-derived undifferentiated osteoblasts are STMN2-, NEBL-, and MGP-expressing cells.

4. A cell preparation according to claim 1, wherein the alveolar bone-derived undifferentiated osteoblasts are contained by being supported on a scaffold.

5. A cell preparation according to claim 1, wherein the cell preparation is for alveolar bone regeneration.

6. A method for producing a cell group that has a bone tissue regenerative capacity,
the method comprising:
a first step of subjecting a collected alveolar bone tissue to enzyme treatment to obtain alveolar bone-derived cells;
a second step of culturing the alveolar bone-derived cells obtained in the first step in a medium that allows for growth of undifferentiated osteoblasts; and
a third step of collecting a cell group grown in the second step.

7. A production method according to claim 6, wherein the medium that allows for growth of undifferentiated osteoblasts used in the second step is a PDGF-containing medium.

8. A production method according to claim 6, wherein the cell group collected in the third step is a group of cells expressing STMN2, NEBL, and MGP.

9. A cell group that has a bone tissue regenerative capacity, and is obtained by culturing undifferentiated osteoblasts collected from alveolar bone.

10. A cell group according to claim 9, wherein the undifferentiated osteoblasts collected from alveolar bone are cultured in a PDGF-containing medium.

11. A cell group according to claim 9, wherein the undifferentiated osteoblasts are STMN2, NEBL, and MGP-expressing cells.

12. A method for treating bone tissue damage,
the method comprising the step of administering the cell preparation of claim 1 to a patient at a bone tissue site involving bone tissue damage.

13. A use of alveolar bone-derived undifferentiated osteoblasts for manufacture of a cell preparation for bone tissue regeneration.

14. A use according to claim 13, wherein the alveolar bone-derived undifferentiated osteoblasts are cells obtained by being cultured in a PDGF-containing medium after enzyme treatment of a collected alveolar bone tissue.

15. A use according to claim 13, wherein the alveolar bone-derived undifferentiated osteoblasts are STMN2-, NEBL-, and MGP-expressing cells.


SEQUENCE LISTING

<110>  OSAKA UNIVERSITY

<120>  Cell preparation for regenerating bone tissue

<130>  P08-133

<150>  JP 2008-210591
<151>  2008-08-19

<160>  18

<170>  PatentIn version 3.1

<210>  1
<211>  21
<212>  DNA
<213>  Artificial

<220>
<223>  Forward primer for OSTERIX

<400>  1
ctgaagaatg ggtggggaag g                                                21


<210>  2
<211>  20
<212>  DNA
<213>  Artificial

<220>
<223>  Reverse primer for OSTERIX

<400>  2
ggcctctgtc ctcctagctc                                                  20


<210>  3
<211>  28
<212>  DNA
<213>  Artificial

<220>
<223>  Forward primer for RUNX2

<400>  3
gaaactcaac agattaacta tcgtttgc                                         28


<210>  4
<211>  27
<212>  DNA
<213>  Artificial

<220>
<223>  Reverse primer for RUNX2

<400>  4
gaatttatca cagatggtcc ctaatgg                                          27


<210>  5
<211>  20
<212>  DNA
<213>  Artificial

<220>
<223>  Forward primer for OSTEOCALCIN

<400>  5
cacactcctc gccctattgg                                                    20


<210>  6
<211>  20
<212>  DNA
<213>  Artificial

<220>
<223>  Reverse primer for OSTEOCALCIN

<400>  6
tgcacctttg ctggactctg                                                    20


<210>  7
<211>  20
<212>  DNA
<213>  Artificial

<220>
<223>  Forward primer for BONE SIALOPROTEIN

<400>  7
cgaatacacg ggcgtcaatg                                                    20


<210>  8
<211>  24
<212>  DNA
<213>  Artificial

<220>
<223>  Reverse primer for BONE SIALOPROTEIN

<400>  8
gtagctgtac tcatcttcat aggc                                               24


<210>  9
<211>  20
<212>  DNA
<213>  Artificial

<220>
<223>  Forward primer for BMP2

<400>  9
ccagaaacga gtgggaaaac                                                    20


<210>  10
<211>  20
<212>  DNA
<213>  Artificial

<220>
<223>  Reverse primer for BMP2

<400>  10
aattcggtga tggaaactgc                                                    20


<210>  11

<211>  25
<212>  DNA
<213>  Artificial

<220>
<223>  Forward primer for GAPDH

<400>  11
aagagcacaa gaggaagaga gagac                                              25


<210>  12
<211>  24
<212>  DNA
<213>  Artificial

<220>
<223>  Reverse primer for GAPDH

<400>  12
ttattgatgg tacatgacaa ggtg                                               24


<210>  13
<211>  20
<212>  DNA
<213>  Artificial

<220>
<223>  Forward primer for STMN2

<400>  13
acgtctgcag gaaaaggaga                                                    20


<210>  14
<211>  20
<212>  DNA
<213>  Artificial

<220>
<223>  Reverse primer for STMN2

<400>  14
acgatgtagt cgccgtctct                                                    20


<210>  15
<211>  20
<212>  DNA
<213>  Artificial

<220>
<223>  Forward primer for NEBL

<400>  15
cattcccaag gctatggcta                                                    20


<210>  16
<211>  20
<212>  DNA
<213>  Artificial

<220>
<223>  Reverse primer for NEBL

<400>  16

acgatgtagt cgccgtctct                                                    20

<210>  17
<211>  20
<212>  DNA
<213>  Artificial

<220>
<223>  Forward primer for MGP

<400>  17
cgcttcctga agtagcgatt                                                    20

<210>  18
<211>  20
<212>  DNA
<213>  Artificial

<220>
<223>  Reverse primer for MGP

<400>  18
ccctcagcag agatggagag                                                    20